(19) Europäisches Patentamt
European Patent Office
Office européen des brevets

(11) Veröffentlichungsnummer: **0 092 814**
**B1**

(12) # EUROPÄISCHE PATENTSCHRIFT

(45) Veröffentlichungstag der Patentschrift: **23.10.85**

(51) Int. Cl.[4]: **C 07 D 451/06**, A 61 K 31/46

(21) Anmeldenummer: **83103935.9**

(22) Anmeldetag: **22.04.83**

(54) **Neue Zwischenprodukte zur Herstellung N-Alkylnorscopine, Verfahren zu ihrer Herstellung und ihre Verwendung.**

(30) Priorität: **26.04.82 DE 3215493**

(43) Veröffentlichungstag der Anmeldung: **02.11.83 Patentblatt 83/44**

(45) Bekanntmachung des Hinweises auf die Patenterteilung: **23.10.85 Patentblatt 85/43**

(84) Benannte Vertragsstaaten: **AT BE CH DE FR GB IT LI LU NL SE**

(56) Entgegenhaltungen:
**CHEMICAL ABSTRACTS, Band 76, 1972, Seite 523, Zusammenfassung Nr. 141129e and Ninth Collective Index, Columbus, Ohio, USA**
**THE JOURNAL OF THE AMERICAN CHEMICAL SOCIETY, Band 79, 1957, Seiten 665-666, Columbus, Ohio, USA J.MEINWALD et al.: "The alkaline hydrolysis of scopolamine methoxymethochloride: A new route to scopine"**

(73) Patentinhaber: **BOEHRINGER INGELHEIM KG, D-6507 Ingelheim am Rhein (DE)**
(84) Benannte Vertragsstaaten: **AT BE CH DE FR GB IT LI LU NL SE**

(73) Patentinhaber: **Boehringer Ingelheim International G.m.b.H, D-6507 Ingelheim am Rhein (DE)**
(84) Benannte Vertragsstaaten: **GB**

(72) Erfinder: **Banholzer, Rolf, Dr., Johann-Calvin-Strasse 11, D-6507 Ingelheim/Rhein (DE)**

**EP 0 092 814 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die Erfindung betrifft neue N-Alkyl-norscopine der allgemeinen Formel

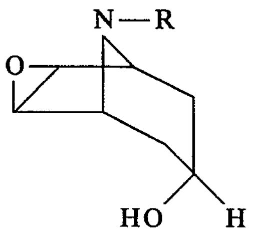

(I)

worin R einen geradkettigen oder verzweigten Alkylrest mit 2—10 Kohlenstoffatomen bedeutet.

Die N-Alkylnorscopine der allgemeinen Formel I sind wertvolle Zwischenprodukte, beispielsweise zur Herstellung neuer Dibenzo-[b,e]-thiepin-11-N-alkyl-norscopinether der allgemeinen Formel II

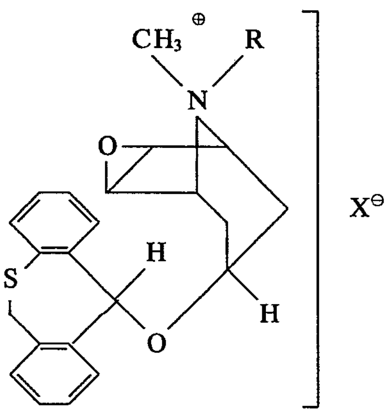

(II)

worin R einen geradkettigen oder verzweigten Alkylrest mit 2—10 Kohlenstoffatomen und $X^{(-)}$ ein pharmakologisch unbedenkliches Anion wie etwa ein Halogenatom oder den Rest einer organischen Sulfonsäure darstellt.

Diese Stoffe sind ihrerseits wertvolle Pharmazeutika mit einem gut ausgewogenen Verhältnis von anticholinerger und antihistaminischer Wirkung. Sie eignen sich daher hervorragend für die Behandlung von Spasmen und obstruktiven Atemwegserkrankungen. Diese Substanzen sind in der Anmeldung EP-A1-0 090 195 näher beschrieben.

Die Weiterverarbeitung der Verbindungen der allgemeinen Formel I zu den N-Alkylnorscopinethern der obenbezeichneten allgemeinen Formel II kann so erfolgen, daß man sie mit Verbindungen der allgemeinen Formel III

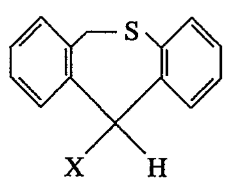

(III)

worin X eine leicht abspaltbare Gruppe (z. B. ein Halogenatom) bedeutet, zu tertiären Verbindungen der allgemeinen Formel IV

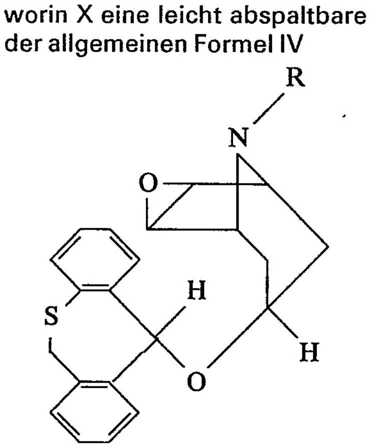

(IV)

worin R die obengenannte Bedeutung hat, umsetzt und letztere wiederum mit üblichen Quaternierungsmitteln der allgemeinen Formel

$CH_3-X$ (V)

worin X die obengenannte Bedeutung hat, in die Quartärsalze der allgemeinen Formel II überführt.

Bezüglich näherer Einzelheiten der Weiterverarbeitung der N-Alkylnorscopine der allgemeinen Formel I wird auf die bereits erwähnte Anmeldung EP-A1-0 090 195 verwiesen.

Die Herstellung der Verbindungen der allgemeinen Formel I erfolgt dabei so, daß man N-Alkylnorscopolamine der allgemeinen Formel VI

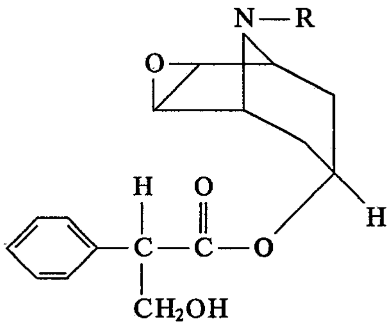

(VI)

bzw. deren Salze mit geeigneten komplexen Metallhydriden (vorzugsweise bei Raumtemperatur oder niedrigeren Temperaturen) in einem Lösungsmittel behandelt. Besonders geeignet sind Metallborhydride und hier wiederum das Natriumborhydrid ($NaBH_4$). Auch Lithiumalanat ist grundsätzlich geeignet, hierbei sollten jedoch zwecks Vermeidung von Nebenreaktionen Temperaturen von 0°C und weniger angewandt werden.

Als Lösungsmittel für die vorbezeichnete Umsetzung kommt vor allem Ethanol in Betracht. Höhere Alkohole und andere organische Lösungsmittel (z. B. Ether) sind weniger günstig, da in ihnen die vorzugsweise als Ausgangsprodukte verwendeten Salze der Verbindungen der allgemeinen Formel VI schwerlöslich sind. Auch Methanol ist nur wenig geeignet, da es beispielsweise Natriumborhydrid schon bei 0°C sehr rasch zersetzt, so daß die Einsatzmenge dieses Reagens' unnötig vergrößert wird. Auch in Wasser kann die Hydrogenolyse der Verbindungen der Formel VI durchgeführt werden, wenn z. B. ein pH von ca. 6—7 eingehalten wird. Die anschließende Isolierung der N-Alkylnorscopine aus Wasser ist jedoch mühsamer als die aus Ethanol.

Die Herstellung der als Ausgangsprodukte benutzten N-Alkylnorscopolamine der allgemeinen Formel VI kann der DE-AS 1 670 048 entnommen werden.

Die folgenden Beispiele erläutern die Erfindung, ohne sie zu beschränken:

## Beispiel 1

### N-Isopropylnorscopin sowie dessen Hydrochlorid

36,8 g (0,1 Mol) N-Isopropylnorscopolamin-hydrochlorid werden in 370 ml Ethanol suspendiert und bei einer Temperatur von 20°C unter ständigem Rühren in sechs Anteilen in Abständen von jeweils 15 Minuten mit 3,78 g (0,1 Mol) Natriumborhydrid versetzt. Man läßt 12 Stunden nachreagieren. Die Hydrogenolyse ist nach dieser Zeit beendet. Bei —5 bis —10°C wird nun unter Stickstoffatmosphäre Chlorwasserstoffgas bis zur sauren Reaktion eingeleitet, die Lösung mit 1,5 Liter Ether frei von Chlorwasserstoff gewaschen. Die nach dem Trocknen fein verriebenen Kristalle suspendiert man in 2 Liter Methylenchlorid, erhitzt zum Sieden und leitet in das siedende Methylenchlorid zur Bildung der Base Ammoniak bis zur völligen Umsetzung ein. Nach Abtrennen der organischen Salze wird das Methylenchlorid unter vermindertem Druck bei 30°C abdestilliert.

Farblose Kristalle (Cyclohexan), Schmelzpunkt 118—119,5°C

Ausbeute: 15,8 g (86,2% d. Th.).

Das Hydrochlorid kann nach üblichen Methoden hergestellt werden.

Farblose Kristalle (Ethanol), Schmelzpunkt 255°C (Zers.), (Umwandlungspunkt ~230°C).

Durch Elementaranalyse und Spektren wird das Vorliegen dieser Verbindung bestätigt.

Beispiel 2

N-Ethylnorscopin-hydrochlorid

35,4 g (0,1 Mol) N-Ethylnorscopolamin-hydrochlorid werden in 350 ml Ethanol suspendiert und bei einer Temperatur von 20°C unter ständigem Rühren in sechs Anteilen in Abständen von jeweils 20 Minuten mit 3,78 g = 0,1 Mol Natriumborhydrid versetzt. Dann kann analog der Synthesevorschrift von Beispiel 1 vorgegangen werden. Aus ethanolischer Lösung erhält man durch Übersättigung mit Ether farblose Kristalle (Ethanol/Ether) vom Schmelzpunkt 139—141°C (Zers.).

Beispiel 3

N-n-Propylnorscopin-hydrochlorid

Aus 36,8 g (0,1 Mol) N-n-Propylnorscopolamin wird mit Hilfe von 3,78 g (= 0,1 Mol) Natriumborhydrid in Ethanol analog der Vorschrift des Beispiels 1 die im Titel genannte Substanz hergestellt.
Farblose Kristalle (aus Ethanol/Ether) vom Schmelzpunkt 241—241,5°C (Zers.).

Beispiel 4

N-n-Butylnorscopin-hydrochlorid

Aus 38,2 g (0,1 Mol) N-n-Butylnorscopolamin wird mit Hilfe von 3,78 g (0,1 Mol) Natriumborhydrid in Ethanol analog der Vorschrift des Beispiels 1 die im Titel genannte Substanz gewonnen.
Farblose Kristalle (aus Acetonitril) vom Schmelzpunkt 198°C.

**Patentansprüche**

1. Neue Verbindungen der allgemeinen Formel

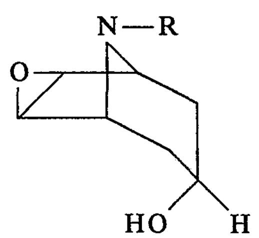

(I)

worin R einen geradkettigen oder verzweigten Alkylrest mit 2—10 Kohlenstoffatomen bedeutet, und deren Säureadditionssalze.

2. Verfahren zur Herstellung von Verbindungen nach Anspruch 1 und deren Salzen, dadurch gekennzeichnet, daß man N-Alkylnorscopolamine der allgemeinen Formel

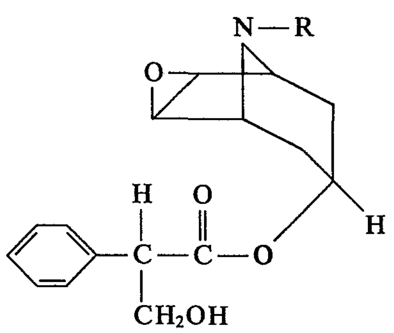

(VI)

worin R die obengenannte Bedeutung hat, bzw. deren Salze mit einem komplexen Hydrid hydrogenolytisch spaltet.

3. Verfahren nach Anspruch 2, dadurch gekennzeichnet, daß man als komplexes Hydrid ein Metallborhydrid einsetzt.

4. Verfahren nach Anspruch 3, dadurch gekennzeichnet, daß man mit Natriumborhydrid bei Raumtemperatur in Ethanol arbeitet.

5. Verwendung der Verbindungen nach Anspruch 1 zur Herstellung von Pharmazeutika.

6. Verfahren zur Herstellung von Verbindungen der Formel

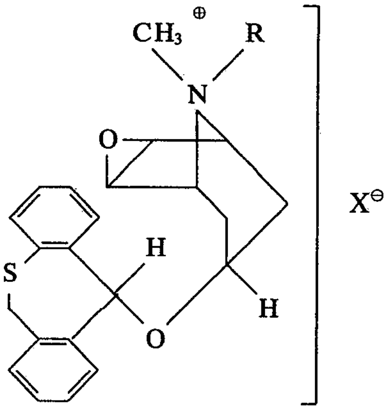

(II)

worin R einen geradkettigen oder verzweigten Alkylrest mit 2 bis 10 Kohlenstoffatomen und $X^-$ ein pharmakologisch unbedenkliches Anion bedeutet, in an sich bekannter Weise, dadurch gekennzeichnet, daß eine Verbindung der Formel

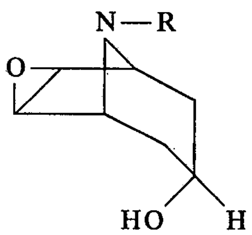

(I)

worin R die oben angegebene Bedeutung hat, mit einer Verbindung der Formel

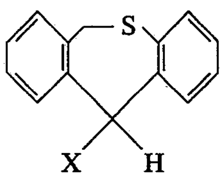

(III)

worin X eine leicht abspaltbare Gruppe, vorzugsweise ein Halogenatom, bedeutet, zu einer Verbindung der Formel

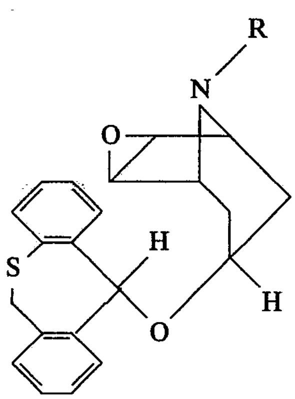

(IV)

worin R die oben angegebene Bedeutung hat, umgesetzt wird und daß anschließend die Verbindung der Formel IV in üblicher Weise mit einer Verbindung

$-CH_3-X$ (V)

worin X die oben genannte Bedeutung hat, quaterniert wird.

**Claims**

1. New compounds of general formula

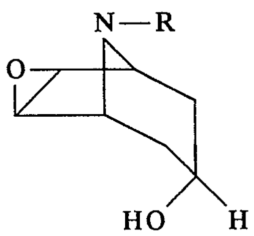

(I)

wherein R represents a straight-chained or branched alkyl group having 2 to 10 carbon atoms, and the acid addition salts thereof.

2. Process for preparing compounds as claimed in claim 1 and the salts thereof, characterised in that N-alkylnorscopolamines of general formula

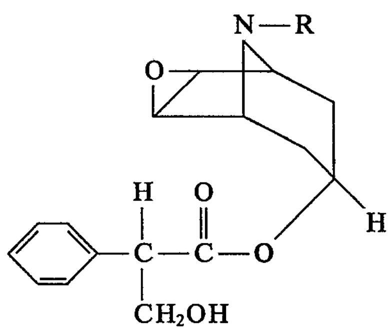

(VI)

wherein R ist as hereinbefore defined, or the salts thereof are cleaved with a complex hydride.

3. Process as claimed in claim 2, characterised in that a metal borohydride is used as the complex hydride.

4. Process as claimed in claim 3, characterised in that the work is done with sodium borohydride at ambient temperature in ethanol.

5. Use of the compounds as claimed in claim 1 for the preparation of pharmaceutical compositions.

6. Process for preparing compounds of formula

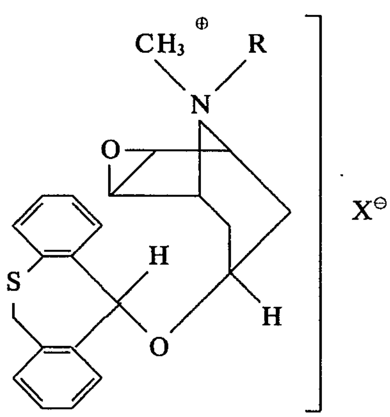

(II)

wherein R represents a straight-chained or branched alkyl group with 2 to 10 carbon atoms and X $^{-}$ represents a pharmacologically harmless anion, in a manner known per se, characterised in that a compund of formula

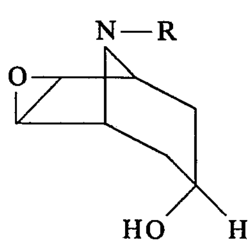

(I)

wherein R is as hereinbefore defined, is reacted with a compound of formula

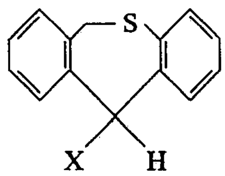

(III)

wherein X represents an easily removable group, preferably a halogen atom, to obtain a compound of formula

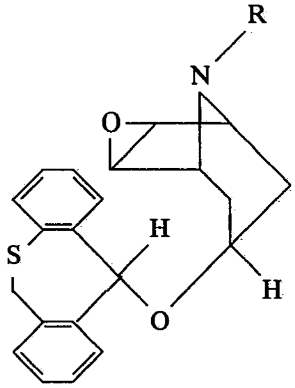

(IV)

wherein R is as hereinbefore defined, and subsequently the compound of formula IV is quaternised in conventional manner with a compound

$CH_3-X$ (V)

wherein X is as hereinbefore defined.

**Revendications**

1. Nouveaux composés de formule générale

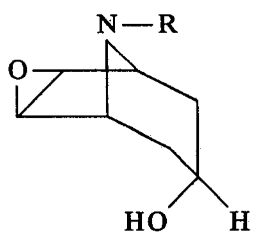

(I)

dans laquelle R représente un radical alcoyle rectiligne ou ramifié avec 2—10 atomes de carbone, et leurs sels d'addition d'acides.

2. Procédé pour la préparation de composés selon la revendication 1 et de leurs sels, caractérisé en ce qu'on clive des N-alcoyl-norscopolyamines de formule générale

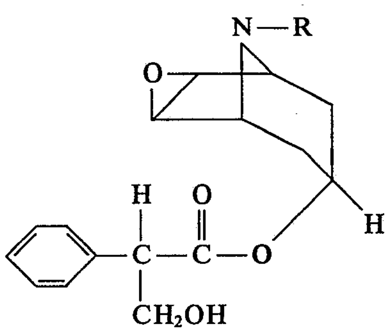

(VI)

dans laquelle R a la signification mentionnée plus haut, ou leurs sels avec un hydrure complexe.

3. Procédé selon la revendication 2, caractérisé en ce que l'on utilise en tant qu'hydrure complexe, un borohydrure métallique.

4. Procédé selon la revendication 3, caractérisé en ce que l'on travaille avec du borohydrure de sodium à la température ambiante dans l'éthanol.

5. Utilisation des composés selon la revendication 1 pour la préparation de produits pharmaceutiques.

6. Procédé pour la préparation de composés de formule

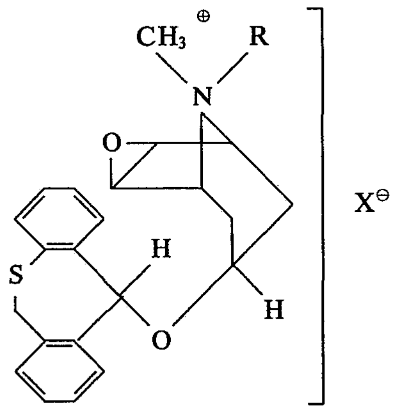

(II)

dans laquelle R représente un radical alcoyle rectiligne ou ramifié avec 2 à 10 atomes de carbone et $X^{(-)}$ un anion pharmacologiquement inoffensif, de manière en soi connue, caractérisé en ce qu'on transforme un composé de formule

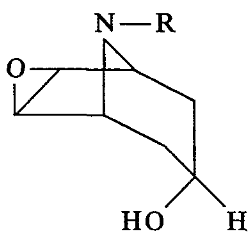

(I)

dans laquelle R a la signification indiquée plus haut, au moyen d'un composé de formule

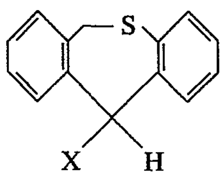

(III)

dans laquelle X représente un groupe facilement clivable, de préférence un atome d'halogène, en un composé de formule

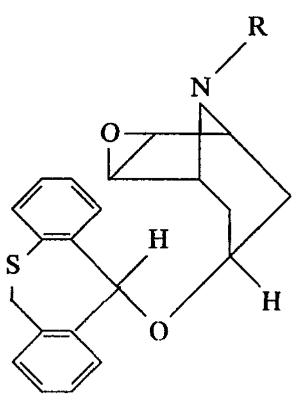

(IV)

dans laquelle R a la signification indiquée plus haut et en ce qu'on quaternise ensuite le composé de formule IV, de manière usuelle, au moyen d'un composé

$$CH_3-X \qquad (V)$$

dans laquelle X a la signification mentionnée plus haut.